# EUROPÄISCHE PATENTSCHRIFT

(11) **EP 2 937 073 B1**
(45) Veröffentlichungstag und Bekanntmachung des Hinweises auf die Patenterteilung: **30.11.2016**
(21) Anmeldenummer: 15162336.0
(22) Anmeldetag: 02.04.2015
(51) Int. Cl.: A61K 8/37, A61Q 19/00, A61K 8/81, A61K 8/06, A61Q 17/04

(54) **EMULSIONSGRUNDLAGE FÜR SONNENSCHUTZMITTEL**
BASIS FOR EMULSION FOR SUN PROTECTING PRODUCTS
BASE D'ÉMULSION POUR LES PRODUITS DE PROTECTION SOLAIREL

(30) Priorität: 23.04.2014 DE 102014207602
(43) Veröffentlichungstag der Anmeldung: 28.10.2015
(73) Patentinhaber: Beiersdorf AG, 20253 Hamburg (DE)
(72) Erfinder: Reiter, Katharina, 22177 Hamburg (DE); Sprock, Sarah, 22297 Hamburg (DE); Pasternak, Anja, 22085 Hamburg (DE)

(56) Entgegenhaltungen:
- EP-A2- 2 712 608
- KR-A- 20070 052 486

## Beschreibung

Die vorliegende Erfindung betrifft eine kosmetische Emulsion enthaltend Polyglyceryl-10 Stearat (INCI Polyglyceryl-10 Stearate) und Natriumpolyacrylat (INCI Sodium Polyacrylate).

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren haben dabei der Zustand und das Aussehen der Haut.

Hautpflegeprodukte bestehen in der Regel aus Emulsionen. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-ÖI-Emulsion (W/O-Emulsion, z. B. Butter) handelt es sich um das umgekehrte Prinzip, wobei der Grundcharakter hier durch das Öl bestimmt wird.

Die Vielzahl an kommerziell erhältlichen kosmetischen Emulsionen darf jedoch nicht darüber hinwegtäuschen, dass diese Zubereitungen des Standes der Technik eine Reihe von Nachteilen aufweisen.

Kosmetische Emulsionen werden häufig mit UV-Lichtschutzfiltern versetzt als Tagespflegeprodukt oder Sonnenschutzmittel eingesetzt. Um hohe Lichtschutzfaktoren (SPF) zu erreichen, müssen dabei relativ große Mengen an UV-Filtern zugesetzt werden. Da in solchen Produkten immer auf ein ausgeglichenes Verhältnis von UV-A und UV-B-Schutz geachtet werden muss, können nur bestimmte UV-Filterkombinationen eingesetzt werden. Der Zusatz der Filter führt dabei regelmäßig dazu, dass die Zubereitung instabil wird. Ganz besondere Stabilitätsprobleme ergeben sich dabei, wenn partikuläre UV-Filter wie beispielsweise Titandioxide eingesetzt werden, denn diese neigen besonders dazu, zu agglomerieren und auszufallen.

Es war daher die Aufgabe der vorliegenden Erfindung, eine stabile kosmetische Emulsion zu entwickeln, in die sich größere Mengen an UV-Lichtschutzfiltern bequem einarbeiten lassen.

Besonders hohe Lichtschutzfaktoren lassen sich mit Öl-in-Wasser-Emulsionen (O/W-Emulsionen) erzielen, bei denen die äußere Phase durch die Wasserphase gebildet wird. Bei diesen Systemen stellt die mikrobielle Stabilität eine besondere Herausforderung dar, weil die äußere Wasserphase den Nährboden für alle Mikroorganismen bildet. Verschärft wird das Problem dadurch, dass insbesondere Sonnenschutzmittel regelmäßig höheren Umgebungstemperaturen ausgesetzt sind (beispielsweise durch die Mitnahme und Anwendung im Sommer in Freibädern oder am Strand), weil höhere Temperaturen das Wachstum der Mikroorganismen beschleunigen. Auf der anderen Seite wünschen die Verbraucher zunehmend Konservierungsmittel-freie Zubereitungen, weil Konservierungsmitteln wie Formaldehyd-Abspaltern, Parabenen etc. negative Wirkungen bei der Anwendung auf den menschlichen Körper unterstellt werden. Auch wenn eine solche Wirkungsweise in der Fachwelt durchaus umstritten ist, besteht der Wunsch, sich eventuellen Risiken erst gar nicht auszusetzen.

Es war daher die Aufgabe der vorliegenden Erfindung, eine kosmetische Emulsion (insbesondere eine O/W-Emulsion) zu entwickeln, die mikrobiell stabil ist ohne, dass die herkömmlichen Konservierungsmittel eingesetzt werden müssen.

Nicht zuletzt müssen kosmetische Emulsionen ein auf das Vorratsbehältnis und die Anwendung abgestimmtes rheologisches Profil aufweisen und sich dabei gleichzeitig angenehm auf der Haut anfühlen. Die Produkte sollen sich leicht und gut dosierbar aus dem Vorratsbehältnis (z.B. Quetschflasche, Tube) entnehmen lassen und einfach und zielgerichtet auf die Haut auftragen lassen. Zur Einstellung des rheologischen Profils (insbesondere einer bestimmten Viskosität) werden nach dem Stand der Technik Verdickungsmittel wie Polyacrylate eingesetzt, die wiederum einen (meist negativen) Einfluss auf das Hautgefühl der Zubereitung haben und auch die Stabilität derselben beeinflussen. Es war daher die Aufgabe der vorliegenden Erfindung, die Nachteile des Standes der Technik zu beseitigen und eine sensorisch angenehme, leicht dosierbare Emulsion zu entwickeln.

Überraschend gelöst werden die Aufgaben durch eine kosmetische Emulsion enthaltend
a) Polyglyceryl-10 Stearat (INCI Polyglyceryl-10 Stearate)
b) Natriumpolyacrylat (INCI Sodium Polyacrylate).

Überraschend gelöst werden die Aufgaben durch ein Verfahren zur Herstellung einer kosmetischen Emulsion, dadurch gekennzeichnet, dass Polyglyceryl-10 Stearate sowie Natriumpolyacrylat (Sodium Polyacrylate) in die erhitzte Fettphase eingearbeitet wird und anschließend die heiße Fettphase mit der Wasserphase vereinigt und homogenisiert wird und durch die nach diesem Verfahren hergestellte kosmetische Emulsion.

Insbesondere war es für den Fachmann überraschend, dass sich die Aufgaben nur dann lösen lassen, wenn das vorneutralisierte Natriumpolyacrylat eingesetzt wird, während der Einsatz von Polyacrylsäure, die erst im Verlauf des Herstellungsprozesses neutralisiert wird, nicht den gewünschten Erfolg bringt. Ferner war es überraschend, dass die Stabilität der Zubereitung durch Natriumpolyacrylat erhöht wird, obwohl diese Verbindung nicht quervernetzt ist und folglich nicht das aus dem Stand der Technik bekannte Gelnetzwerk ausbilden kann.

Zwar kennt der Stand der Technik das koreanische Patent mit der Anmeldenummer 10-2005-0110176 (Veröffentlichungsnummer: Kr10-2007-0052486) sowie die EP2712608, doch konnten diese Schriften nicht den Weg zur vorliegenden Erfindung weisen.

Im Rahmen der vorliegenden Offenbarung beziehen sich die Formulierungen "erfindungsgemäß", erfindungsgemäß vorteilhaft" etc. und "Emulsion", "Zubereitung" etc. immer sowohl auf die erfindungsgemäße Emulsion, das erfindungsgemäße Verfahren und das erfindungsgemäße Verfahrensprodukt.

Es ist dabei erfindungsgemäß von Vorteil, wenn die Zubereitung Polyglyceryl-10 Stearat in einer Konzentration von 0,4 bis 1,5 Gewichts-%, bezogen auf das Gesamtgewicht der Emulsion, enthält.

Erfindungsgemäß bevorzugt ist es, wenn die Zubereitung Polyglyceryl-10 Stearat in einer Konzentration von 0,5 bis 1,2 Gewichts-%, bezogen auf das Gesamtgewicht der Emulsion, enthält.

Erfindungsgemäß bevorzugt wird als Polyglyceryl-10 Stearat das Produkt mit der CAS No.: 37349-34-1 eingesetzt. Erfindungsgemäß vorteilhaft ist es, wenn das Polyglyceryl-10 Stearat einen HLB-Wert von 12 bis 14 (und bevorzugt von 13) aufweist. Ein derartiges Polyglyceryl-10 Stearat kann beispielsweise unter dem Handelsnamen Polyaldo^{®} 10-1-S KFG bei der Firma Lonza bezogen werden.

Es ist erfindungsgemäß vorteilhaft, wenn die Zubereitung Natriumpolyacrylat in einer Konzentration von 0,05 bis 0,8 Gewichts-%, bezogen auf das Gesamtgewicht der Emulsion, enthält.

Erfindungsgemäß bevorzugt ist es, wenn die Zubereitung Natriumpolyacrylat in einer Konzentration von 0,05 bis 0,5 Gewichts-%, bezogen auf das Gesamtgewicht der Emulsion, enthält.

Es ist erfindungsgemäß von Vorteil, wenn das eingesetzte Natriumpolyacrylat einen pH-Wert von 5,5 bis 6,5 (gemessen bei 20 °C, in einer Konzentration von 10g/l Wasser) aufweist.

Außerdem ist es erfindungsgemäß von Vorteil, wenn das eingesetzte Natriumpolyacrylat als Rohstoff eine Dichte von 0,50 bis 0,70 g/cm³ (bei 20 °C, vor der Einarbeitung) aufweist.

Eine erfindungsgemäß bevorzugte Ausführungsform ist das Natriumpolyacrylat mit der CAS No.:9003-04-7. Ein derartiges Natriumpolyacrylat kann beispielsweise unter dem Handelsnamen Cosmedia^{®} SP bei der Firma BASF bezogen werden.

Es ist erfindungsgemäß vorteilhaft, wenn die erfindungsgemäße Zubereitung in Form einer O/W-Emulsion vorliegt.

Erfindungsgemäß vorteilhafte Ausführungsformen der vorliegenden Erfindung sind dadurch gekennzeichnet, dass die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-meth-oxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Es ist erfindungsgemäß vorteilhaft, wenn die erfindungsgemäße Zubereitung Homomenthylsalicylat (Homosalate) und/oder Ethylhexylsalicylat enthält, wobei eine Kombination beider UV-Filter erfindungsgemäß bevorzugt ist.

Erfindungsgemäß besonders bevorzugte Emulsionen werden durch eine Kombination aus Titandioxid, Homomenthylsalicylat (Homosalate) und Ethylhexylsalicylat erhalten.

Erfindungsgemäß vorteilhafte Ausführungsformen sind dadurch gekennzeichnet, dass die Zubereitung Homomenthylsalicylat (Homosalate) und Ethylhexylsalicylat in einer Gesamtmenge von 7.5 bis 17 Gewichts-%, bezogen auf das Gesamtgewicht der Zubereitung, enthält (wobei sich die Gesamtmenge auf die Einzelkomponente bezieht, falls nur einer der beiden UV-Filter in der Zubereitung vorhanden ist).

Erfindungsgemäß bevorzugte Ausführungsformen sind dadurch gekennzeichnet, dass die Zubereitung Homomenthylsalicylat (Homosalate) und Ethylhexylsalicylat in einer Gesamtmenge von 10 bis 15 Gewichts-%, bezogen auf das Gesamtgewicht der Zubereitung, enthält (wobei sich die Gesamtmenge auf die Einzelkomponente bezieht, falls nur einer der beiden UV-Filter in der Zubereitung vorhanden ist).

Es ist erfindungsgemäß vorteilhaft, wenn die erfindungsgemäße Emulsion Ethanol enthält.

In einem solchen Falle beträgt die erfindungsgemäß bevorzugte Konzentration von Ethanol in der Zubereitung von 2 bis 10 Gewichts-%, bezogen auf das Gesamtgewicht der Zubereitung.

Es ist erfindungsgemäß besonders bevorzugt, wenn die Zubereitung frei ist von Propyl- und Butylparaben, 3-lod-2-propinylbutylcarbamat, 3-(4-Methylbenzyliden)-campher und 2-Hydroxy-4-methoxybenzophenon (Oxybenzon).

Erfindungsgemäß vorteilhafte Ausführungsformen können dadurch erhalten werden, dass die Zubereitung Ethylhexylglycerin, Polyglyceryl-2 Caprate, Propylenglycol, Butylenglycol, 2-Methylpropan-1,3-diol, 1,2-Pentandiol, 1,2-Hexandiol, 1,2-Octandiol und/oder 1,2-Decandiol enthält.

Außerdem ist es erfindungsgemäß vorteilhaft, wenn die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, ß-Alanin, Bisabolol, Vitamin C, Panthenol und/oder Licochalcon A, enthält.

Die erfindungsgemäße Zubereitung kann ferner vorteilshaft Glycerin enthalten. In einem solchen Falle ist eine Glycerin-Konzentration von 4 bis 10 Gewichts-%, bezogen auf das Gesamtgewicht der Zubereitung, erfindungsgemäß vorteilhaft.

Die Ölphase der erfindungsgemäßen Emulsion kann darüber hinaus noch weitere Öl-, Fett- und Wachskomponenten enthalten, beispielsweise polaren Öle aus der Gruppe der Lecithine oder Verbindungen wie z. B. Cocoglycerid, Capryl/Caprinsäure Triglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr. Auch Verbindungen wie Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat können eingesetzt werden. Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Ferner kann die Ölphase vorteilhaft gewählt werden aus der Gruppe der Dialkylether und Dialkylcarbonate, vorteilhaft sind z. B. Dicaprylylether (*Cetiol OE)* und/oder Dicaprylylcarbonat, beispielsweise das unter der Handelsbezeichnung *Cetiol* CC bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid gewählt werden. Es ist insbesondere vorteilhaft, wenn die Ölphase der erfindungsgemäßen Formulierungen einen Gehalt an C₁₂₋₁₅-Alkylbenzoat aufweist.

Vorteilhafte Ölkomponenten sind ferner z. B. Isopropylpalmitat, Myristylmyristat, Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Erfindungsgemäß vorteilhafte Ausführungsformen der vorliegenden Erfindung sind auch dadurch gekennzeichnet, dass die Emulsion Dimethicone und/oder Cyclomethicon enthält.

Als polymeren Filmbildner zur Erhöhung der Wasserfestigkeit kann die erfindungsgemäße Zubereitung erfindungsgemäß vorteilhaft Vinylpyrrolidon/Hexadecen Copolymer enthalten. Darüber hinaus ist der Zusatz von Xanthangummi und/oder Tapiokastärke erfindungsgemäß vorteilhaft.

Die erfindungsgemäße Zubereitung kann insbesondere vorteilhaft als Tagespflegeprodukt oder Sonnenschutzmittel eingesetzt werden.

### Vergleichsversuch

Mit dem folgenden Versuch konnte der erfindungsgemäße Effekt beispielhaft belegt werden: Es wurden die folgenden Rezepturen hergestellt und bei Raumtemperatur gelagert. Der Einsatz von nicht vorneutralisierten Cabomeren und Acrylates/C10-30 Alkyl Acrylate Crosspolymeren führte zu Instabilitäten. Diese äußerten sich in Ölabscheidung auf der Oberfläche innerhalb eines Tages.

| Rezeptur | 1 | 2 | 3 |
|---|---|---|---|
| Homosalate | 9.5 | 9.5 | 9.5 |
| Octocrylene | 8 | 8 | 8 |
| Ethylhexyl Salicylate | 4.75 | 4.75 | 4.75 |
| Bis-Ethylhexyloxyphenol Methoxyphenyl Triazine | 4.5 | 4.5 | 4.5 |
| Butyl Methoxydibenzoylmethane | 4.75 | 4.75 | 4.75 |
| Phenylbenzimidazole Sulfonic Acid | 1 | 1 | 1 |
| Glyceryl Stearate | 1 | 1 | 1 |
| Hydrogenated Coco-Glycerides | 1 | 1 | 1 |
| Polyglyceryl-10 Stearate | 0.8 | 0.8 | 0.8 |
| Xanthan Gum | 0.4 | 0.4 | 0.4 |
| Sodium Polyacrylate | 0.125 | | |
| Acrylates/C10-30 Alkyl Acrylate Crosspolymer (Carbopol 1382) | | 0.2 | |
| Carbomer (Carbopol 980) | | | 0.2 |
| Aqua | 41.285 | 41.09 | 41.07 |
| Alcohol Denat. + Aqua | 7 | 7 | 7 |
| Ethylhexylglycerin | 0.15 | 0.15 | 0.15 |
| Polyglyceryl-2 Caprate | 0.3 | 0.3 | 0.3 |
| Methylpropanediol | 1 | 1 | 1 |
| Aqua + Trisodium EDTA | 1 | 1 | 1 |
| Silica Dimethyl Silylate | 1.5 | 1.5 | 1.5 |
| Tapioca Starch + Aqua | 1 | 1 | 1 |
| VP/Hexadecene Copolymer | 0.5 | 0.5 | 0.5 |
| Glycerin + Aqua | 8.6 | 8.6 | 8.6 |
| Tocopheryl Acetate | 0.06 | 0.06 | 0.06 |
| Panthenol + Aqua | 1.4 | 1.4 | 1.4 |
| Aqua + Sodium Hydroxide | 0.38 | 0.5 | 0.52 |

Rezeptur 1 mit Sodium Polyacrylate ist auch nach zwei Wochen noch sehr stabil, während Ansatz 2 und 3 bereits nach einem Tag Instabilitäten zeigen.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **A** | **Rezeptur 1** | **Rezeptur 2** |
|---|---|---|
| Homosalate | 9 | 9.5 |
| Octocrylene | 8 | 8 |
| Ethylhexyl Salicylate | 4.5 | 4.75 |
| Butyl Methoxydibenzoylmethane | 4.5 | 4.75 |
| Bis-Ethylhexyloxyphenol Methoxyphenyl Triazine | 0 | 4.5 |
| Polyglyceryl-10 Stearate | 0.6 | 0.8 |
| Glyceryl Stearate | 1 | 1 |
| Hydrogenated Coco-Glycerides | 1 | 1 |
| Caprylic/Capric Triglyceride | 3 | 0 |
| Silica Dimethyl Silylate | 1 | 1.5 |
| Polyglyceryl-2 Caprate | 0.3 | 0.3 |
| Tocopheryl Acetate | 0.06 | 0.06 |
| VP/Hexadecene Copolymer | 0.5 | 0.5 |
| | | |
| **B** | | |
| Xanthan Gum | 0.4 | 0.4 |
| Sodium Polyacrylate | 0.1 | 0.125 |
| Titanium Dioxide (nano) (+ Silica + Methicone + Aqua) | 4 | 0 |
| | | |
| **C** | | |
| Glycerin + Aqua | 8.6 | 8.6 |
| Methylpropanediol | 1 | 1 |
| Aqua + Trisodium EDTA | 1 | 1 |
| Aqua | add to 100 | add to 100 |
| | | |
| **D** | | |
| Tapioca Starch + Aqua | 1 | 1 |
| Alcohol Denat. + Aqua | 7 | 7 |
| Ethylhexylglycerin | 0.15 | 0.15 |
| Panthenol + Aqua | 0 | 1.4 |

Phase A wird bei ca. 85°C aufgeschmolzen. Wenn die Temperatur erreicht ist, wird Phase B in Phase A dispergiert und das Gemisch wieder auf 85°C temperiert. Phase C wird auf 75°C erhitzt. Wenn die Phasen A+B und C die jeweiligen Temperaturen erreicht haben erfolgt unter schnellem Rühren die Phasenvereinigung. Nach ca. 3-4 min Rühren (oder bei 65°C) erfolgt die Heißhomogenisierung. Anschließend wird die Emulsion stetig bei mittlerer Umdrehung gerührt, bis sie sich auf ca. 30°C abgekühlt hat. Dann wird Phase D hinzugegeben, Nach ca. 15 min Rühren wird die Emulsion erneut homogenisiert.

## Patentansprüche

1. Kosmetische Emulsion enthaltend
a) Polyglyceryl-10 Stearat (INCI Polyglyceryl-10 Stearate)
b) Natriumpolyacrylat (INCI Sodium Polyacrylate).

2. Emulsion nach Anspruch 1, **dadurch gekennzeichnet, dass** die Zubereitung Polyglyceryl-10 Stearat in einer Konzentration von 0,4 bis 1,5 Gewichts-%, bezogen auf das Gesamtgewicht der Emulsion, enthält.

3. Emulsion nach einem der vorhergehenden Ansprüche, **dadurch gekennzeichnet, dass** die Zubereitung Natriumpolyacrylat in einer Konzentration von 0,05 bis 0,8 Gewichts-%, bezogen auf das Gesamtgewicht der Emulsion, enthält.

4. Emulsion nach einem der vorhergehenden Ansprüche, **dadurch gekennzeichnet, dass** das eingesetzte Natriumpolyacrylat einen pH-Wert von 5,5 bis 6,5 (gemessen bei 20 °C, in einer Konzentration von 10g/l) aufweist.

5. Emulsion nach einem der vorhergehenden Ansprüche, **dadurch gekennzeichnet, dass** das eingesetzte Natriumpolyacrylat als Rohstoff eine Dichte von 0,50 bis 0,70 g/cm³ (bei 20 °C, vor der Einarbeitung) aufweist.

6. Emulsion nach einem der vorhergehenden Ansprüche, **dadurch gekennzeichnet, dass** die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)-benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat (Homosalate); 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethylhexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

7. Emulsion nach einem der vorhergehenden Ansprüche, **dadurch gekennzeichnet, dass** die Emulsion eine Kombination aus Titandioxid, Homomenthylsalicylat (Homosalate) und Ethylhexylsalicylat enthält.

8. Emulsion nach einem der vorhergehenden Ansprüche, **dadurch gekennzeichnet, dass** die Zubereitung Homomenthylsalicylat (Homosalate) und Ethylhexylsalicylat in einer Gesamtmenge von 7,5 bis 17 Gewichts-%, bezogen auf das Gesamtgewicht der Zubereitung, enthält.

9. Emulsion nach einem der vorhergehenden Ansprüche, **dadurch gekennzeichnet, dass** die Emulsion Ethanol enthält.

10. Emulsion nach einem der vorhergehenden Ansprüche, **dadurch gekennzeichnet, dass** die Zubereitung frei ist von Propyl- und Butylparaben, 3-lod-2-propinylbutylcarbamat, 3-(4-Methylbenzyliden)-campher und 2-Hydroxy-4-methoxybenzophenon (Oxybenzon).

11. Emulsion nach einem der vorhergehenden Ansprüche, **dadurch gekennzeichnet, dass** die Zubereitung Ethylhexylglycerin, Polyglyceryl-2 Caprate, Propylenglycol, Butylenglycol, 2-Methylpropan-1,3-diol, 1,2-Pentandiol, 1,2-Hexandiol, 1,2-Octandiol und/oder 1,2-Decandiol enthält.

12. Emulsion nach einem der vorhergehenden Ansprüche, **dadurch gekennzeichnet, dass** , die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, ß-Alanin, Bisabolol, Vitamin C, Panthenol und/oder Licochalcon A, enthält.

13. Verfahren zur Herstellung einer kosmetischen Emulsion nach einem der vorhergehenden Ansprüche, **dadurch gekennzeichnet, dass** Polyglyceryl-10 Stearate sowie Natriumpolyacrylat (Sodium Polyacrylate) in die erhitzte Fettphase eingearbeitet wird und anschließend die heiße Fettphase mit der Wasserphase vereinigt und homogenisiert wird.

## Claims

1. Cosmetic emulsion comprising
a) polyglyceryl-10 stearate (INCI Polyglyceryl-10 Stearate)
b) sodium polyacrylate (INCI Sodium Polyacrylate).

2. Emulsion according to Claim 1, **characterized in that** the preparation comprises polyglyceryl-10 stearate at a concentration of 0.4 to 1.5% by weight, based on the total weight of the emulsion.

3. Emulsion according to either of the preceding claims, **characterized in that** the preparation comprises sodium polyacrylate at a concentration of 0.05 to 0.8% by weight, based on the total weight of the emulsion.

4. Emulsion according to any of the preceding claims, **characterized in that** the sodium polyacrylate used has a pH of 5.5 to 6.5 (measured at 20°C, at a concentration of 10g/l).

5. Emulsion according to any of the preceding claims, **characterized in that** the sodium polyacrylate used as raw material has a density of 0.50 to 0.70 g/cm³ (at 20°C, prior to incorporation).

6. Emulsion according to any of the preceding claims, **characterized in that** the preparation comprises one or more UV filters selected from the group of compounds comprising 2-phenylbenzimidazole-5-sulphonic acid and/or salts thereof; phenylene-1,4-bis(2-benzimidazyl)-3,3',5,5'-tetrasulphonic acid salts; 1,4-di(2-oxo-10-sulpho-3-bornylidenemethyl)benzene and salts thereof; 4-(2-oxo-3-bornylidenemethyl)benzenesulphonic acid salts; 2-methyl-5-(2-oxo-3-bornylidenemethyl)sulphonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulphonic acid; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; 4-(tert-butyl)-4'-methoxydibenzoylmethane; homomenthyl salicylate (homosalate); 2-ethylhexyl 2-hydroxybenzoate; dimethicodiethylbenzalmalonate; 3-(4-(2,2-bisethoxycarbonylvinyl)phenoxy)propenyl)methoxysiloxane / dimethylsiloxane copolymer; 4-(tert-butyl)-4'-methoxydibenzoylmethane; hexyl 2-(4'-diethylamino-2'-hydroxybenzoyl)benzoate; dioctylbutylamidotriazone (INCI: Diethylhexyl Butamido Triazone); 2,4-bis[5-1 (dimethylpropyl)benzoxazol-2-yl-(4-phenyl)imino]-6-(2-ethylhexyl)imino-1,3,5-triazine with (CAS No. 288254-16-0); 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); tris(2-ethylhexyl) 4,4',4"-(1,3,5-triazine-2,4,6-triyltriimino) trisbenzoate (also: 2,4,6-tris[anilino(p-carbo-2'-ethyl-1'-hexyloxy)]-1,3,5-triazine (INCI: Ethylhexyl Triazone); 2,4,6-tribiphenyl-4-yl-1,3,5-triazine; merocyanine; titanium dioxide; zinc oxide.

7. Emulsion according to any of the preceding claims, **characterized in that** the emulsion comprises a combination of titanium dioxide, homomenthyl salicylate (homosalate) and ethylhexyl salicylate.

8. Emulsion according to any of the preceding claims, **characterized in that** the preparation comprises homomenthyl salicylate (homosalate) and ethylhexyl salicylate in a total amount of 7.5 to 17% by weight, based on the total weight of the preparation.

9. Emulsion according to any of the preceding claims, **characterized in that** the emulsion comprises ethanol.

10. Emulsion according to any of the preceding claims, **characterized in that** the preparation is free of propylparaben and butylparaben, 3-iodo-2-propynyl butylcarbamate, 3-(4-methylbenzylidene)camphor and 2-hydroxy-4-methoxybenzophenone (oxybenzone).

11. Emulsion according to any of the preceding claims, **characterized in that** the preparation comprises ethylhexylglycerol, polyglyceryl-2 caprate, propylene glycol, butylene glycol, 2-methylpropane-1,3-diol, 1,2-pentanediol, 1,2-hexanediol, 1,2-octanediol and/or 1,2-decanediol.

12. Emulsion according to any of the preceding claims, **characterized in that** the preparation comprises one or more active ingredients selected from the group of compounds glycyrrhetic acid, urea, arctiin, alpha-lipoic acid, folic acid, phytoene, D-biotin, coenzyme Q10, alpha-glucosylrutin, carnitine, carnosine, caffeine, natural and/or synthetic isoflavonoids, glycerylglucose, creatine, creatinine, taurine, tocopherol, tocopherol acetate, β-alanine, bisabolol, vitamin C, panthenol and/or licochalcone A.

13. Method for preparing a cosmetic emulsion according to any of the preceding claims, **characterized in that** polyglyceryl-10 stearate and sodium polyacrylate (Sodium Polyacrylate) is incorporated into the heated fat phase and subsequently the hot fat phase is combined and homogenized with the water phase.

## Revendications

1. Emulsion cosmétique, contenant
a) du poly(stéarate de glycéryle-10) (INCI Polyglyceryl-10 Stearate),
b) du poly(acrylate de sodium) (INCI Sodium Polyacrylate).

2. Emulsion selon la revendication 1, **caractérisée en ce que** la préparation contient du poly(stéarate de glycéryle-10) à une concentration de 0,4 à 1,5 % en poids, par rapport au poids total de l'émulsion.

3. Emulsion selon l'une des revendications précédentes, **caractérisée en ce que** la préparation contient du poly(acrylate de sodium) à une concentration de 0,05 à 0,8 % en poids, par rapport au poids total de l'émulsion.

4. Emulsion selon l'une des revendications précédentes, **caractérisée en ce que** le poly(acrylate de sodium) utilisé présente un pH de 5,5 à 6,5 (mesuré à 20°C à une concentration de 10 g/l).

5. Emulsion selon l'une des revendications précédentes, **caractérisée en ce que** le poly(acrylate de sodium) utilisé présente en tant que matière première une masse volumique de 0,50 à 0,70 g/cm³ (à 20°C, avant incorporation).

6. Emulsion selon l'une des revendications précédentes, **caractérisée en ce que** la préparation contient un ou plusieurs filtres UV, choisis dans le groupe des composés acide 2-phénylbenzimidazole-5-sulfonique et/ou ses sels ; sels de l'acide phénylène-1,4-bis(2-benzimidazyl)-3,3'-5,5'-tétrasulfonique ; 1,4-di(2-oxo-10-sulfo-3-bornylidèneméthyl)-benzène et ses sels ; sels de l'acide 4-(2-oxo-3-bornylidèneméthyl)-benzènesulfonique ; sels de l'acide 2-méthyl-5-(2-oxo-3-bornylidèneméthyl)sulfonique ; 2,2'-méthylène-bis(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidène-camphre ; salicylate d'éthylhexyle ; acide téréphtalidènedicamphosulfonique ; acrylate de 2-éthylhexyl-2-cyano-3,3-diphényle ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; 4-(tert-butyl)-4'-méthoxydibenzoylméthane ; salicylate d'homomenthyle (homosalate) ; 2-hydroxybenzoate de 2-éthylhexyle ; malonate de diméthicodiéthylbenzal ; copolymère 3-(4-(2,2-bis-éthoxycarbonylvinyl)-phénoxy)propényl)-méthoxysiloxane / diméthylsiloxane ; 4-(tert-butyl)-4'-méthoxydibenzoylméthane ; ester hexylique de l'acide 2-(4'-diéthylamino-2'-hydroxybenzoyl)-benzoïque ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine portant le numéro CAS 288254-16-0) ; 2,4-bis{[4-(2-éthyl-hexyloxy)-2-hydroxy]phényl}-6-(4-méthoxyphényl)-1,3,5-triazine (INCI : Bis-Ethylhexyloxyphenol Methoxyphenyl Triazin) ; ester tris(2-éthylhexylique) de l'acide 4,4',4"-(1,3,5-triazine-2,4,6-triyltriimino)-tris-benzoique (aussi : 2,4,6-tris[anilino-(p-carbo-2'-éthyl-1'-hexyloxy)]-1,3,5-triazine (INCI : Ethylhexyl Triazone) ; 2,4,6-tribiphényl-4-yl-1,3,5-triazine ; mérocyanine ; dioxyde de titane ; oxyde de zinc.

7. Emulsion selon l'une des revendications précédentes, **caractérisée en ce que** l'émulsion contient une combinaison de dioxyde de titane, de salicylate d'homomenthyle (Homosalate) et de salicylate d'éthylhexyle.

8. Emulsion selon l'une des revendications précédentes, **caractérisée en ce que** la préparation contient du salicylate d'homomenthyle (Homosalate) et du salicylate d'éthylhexyle en une quantité totale de 7,5 à 17 % en poids par rapport au poids total de la composition.

9. Emulsion selon l'une des revendications précédentes, **caractérisée en ce que** l'émulsion contient de l'éthanol.

10. Emulsion selon l'une des revendications précédentes, **caractérisée en ce que** la préparation est exempte de propyl- et de butylparaben, de carbamate de 3-iodo-2-propynylbutyle, de 3-(4-méthylbenzylidène)-camphre et de 2-hydroxy-4-méthoxybenzophénone (Oxybenzone).

11. Emulsion selon l'une des revendications précédentes, **caractérisée en ce que** la préparation contient de l'éthylhexylglycérol, du poly(caprate de glycéryle-2), du propylèneglycol, du butylèneglycol, du 2-méthylpropane-1,3-diol, du 1,2-pentanediol, du 1,2-hexanediol, du 1,2-octanediol et/ou du 1,2-décanediol.

12. Emulsion selon l'une des revendications précédentes, **caractérisée en ce que** la préparation contient une ou plusieurs matières actives choisies dans le groupe des composés acide glycyrrétinique, urée, arctiine, acide alpha-lipoïque, acide folique, phytoène, D-biotine, coenzyme Q10, alpha-glucosylrutine, carnitine, carnosine, caféine, isoflavonoïdes naturels et/ou synthétiques, glycérylglucose, créatine, créatinine, taurine, tocophérol, acétate de tocophérol, □-alanine, bisabolol, vitamine C, panthénol et/ou licochalcone A.

13. Procédé de préparation d'une émulsion cosmétique selon l'une des revendications précédentes, **caractérisé en ce qu'**on incorpore dans la phase grasse chauffée du poly(stéarate de glycéryle-10) ainsi que du poly(acrylate de sodium) (sodium polyacrylate), puis que l'on combine la phase grasse chaude à la phase aqueuse, et on l'homogénéise.
